# EUROPEAN PATENT APPLICATION

(11) **EP 1 920 754 A2**
(43) Date of publication of application: **14.05.2008**
(21) Application number: 07119437.7
(22) Date of filing: 26.10.2007
(51) Int. Cl.: A61K 8/04, A61K 8/81, A61K 8/91, A61Q 1/10

(54) **Detackified compositions**

(30) Priority: 08.11.2006 US 594197
(71) Applicant: L'ORÉAL, 75008 Paris (FR)
(72) Inventor: Keene, Diane, Burlington, NJ 08016 (US); Pavel, Florentina, M., Perth Amboy, NJ 08861 (US); Kljuic, Ana, New York, NY 10011 (US); Feng, Sue, Edison, NJ 08820 (US)
(74) Representative: Le Coupanec, Pascale A.M.P.

(57) **Abstract**

The present invention relates to compositions including a detackifying effective amount of at least one polyurethane/poly(meth)acrylate graft copolymer as well as to methods of using such compositions and to kits comprising such compositions.

## Description

### FIELD OF THE INVENTION

The present invention generally relates to compositions comprising a detackifying effective amount of at least one polyurethane/poly(meth)acrylate graft copolymer as well as to methods of using such compositions and to kits comprising such compositions. Such compositions possess improved properties and characteristics such as, for example, decreased tackiness, improved feeling and spreadability upon application, and decreased clumping of keratin materials (for example, eyelashes) upon application.

### BACKGROUND OF THE INVENTION

Many cosmetic compositions have been developed for longer wear and transfer resistance properties. This is typically accomplished by the use of ingredients that form a film after application. Such compositions generally contain volatile solvents, which evaporate on contact with the skin or eyelashes, leaving behind a layer comprising waxes and/or resins, pigments, fillers, and actives. However, these compositions can be uncomfortable for the wearer as the composition remains on the skin or eyelashes as a brittle or non-flexible film. Such compositions may not be pliable or soft, and they may not be comfortable to wear. There may also be a tendency for such compositions to flake off because of poor adherence to the skin or eyelashes.

Furthermore, such compositions have a tendency to be tacky (that is, sticky) owing to the presence of tacky ingredients in the compositions (particularly tacky film forming agents), resulting in poor application, spreadability and wear characteristics.

Thus, there remains a need for improved cosmetic compositions, particularly long-wearing cosmetic compositions, which transfer little or not at all, i.e., "transfer-free" or transfer resistant compositions which also possess good cosmetic properties such as pliability, comfort and reduced tackiness.

Accordingly, one aspect of the present invention is a care and/or makeup and/or treatment composition for keratin materials such as eyelashes which is able to address or overcome at least one of the aforementioned problems with the prior art compositions.

### SUMMARY OF THE INVENTION

The present invention relates to compositions comprising a detackifying effective amount of at least one polyurethane/poly(meth)acrylate graft copolymer.

The present invention also relates to compositions for eyelashes such as mascaras, topcoats and basecoats comprising a detackifying effective amount of at least one polyurethane/poly(meth)acrylate graft copolymer.

The present invention relates to compositions comprising a detackifying effective amount of at least one polyurethane/poly(meth)acrylate graft copolymer, wherein the compositions are wax-free or substantially wax-free.

The present invention also relates to methods of improving performance properties of compositions such as, for example, transfer-resistance, long-wear and/or waterproofing, comprising adding at least one polyurethane/poly(meth)acrylate graft copolymer to the composition.

The present invention further relates to methods for detackifying a composition comprising adding at least one polyurethane/poly(meth)acrylate graft copolymer to the composition in an amount sufficient to reduce the tackiness of the composition.

The present invention also relates to methods of increasing eyelash volume and/or length comprising applying to eyelashes an eyelash volume- and/or length-increasing effective amount of a composition comprising at least one polyurethane/poly(meth)acrylate graft copolymer.

The present invention also relates to methods of curling eyelashes comprising applying to eyelashes an eyelash curling effective amount of a composition comprising at least one polyurethane/poly(meth)acrylate graft copolymer.

The present invention further relates to methods of making-up keratin materials such as skin, lips or eyelashes comprising applying a keratin material making-up effective amount of a composition comprising at least one polyurethane/poly(meth)acrylate graft copolymer to keratin materials in need of such making-up.

The present invention also relates to methods of treating or caring for keratin materials such as skin, lips or eyelashes by applying compositions of the present invention to the keratin materials in an amount sufficient to treat and/or care for the keratin materials.

The present invention further relates to methods of enhancing the appearance of keratin materials such as skin, lips or eyelashes by applying compositions of the present invention to the keratin materials in an amount sufficient to enhance the appearance of the keratin materials.

The present invention also relates to methods of improving transfer-resistance and/or long-wear properties of a composition for a keratin material comprising adding at least one polyurethane/poly(meth)acrylate graft copolymer to the composition in an amount sufficient to improve the transfer-resistance and/or long-wear properties of the composition.

The present invention also relates to methods of preparing a composition comprising at least one polyurethane/poly(meth)acrylate graft copolymer and at least one tackifying film forming agent comprising dispersing either the at least one polyurethane/poly(meth)acrylate graft copolymer or the at least one tackifying film forming agent in a composition, and then adding the other ingredient to the composition.

The present invention also relates to compositions comprising at least one polyurethane/poly(meth)acrylate graft copolymer and at least one tackifying film forming agent, wherein the polyurethane/poly(meth)acrylate graft copolymer is present in an amount sufficient to reduce the tackiness of the composition.

According to an embodiment, a composition of the invention is an eyelash composition.

The present invention alos related to compositions comprising an aqueous dispersion of polymer particles selected from the group consisting of ethylene.styrene/acrylates copolymers, acrylates/ammonium methacrylate copolymers, and mixtures thereof, and at least one tackifying film forming agent, wherein the aqueous dispersion of polymer particles is present in an amount sufficient to reduce the tackiness of the compositionIt is to be understood that both the foregoing general description and the following detailed description are exemplary and explanatory only, and are not restrictive of the invention.

### DETAILED DESCRIPTION OF THE INVENTION

As used herein, the expression "at least one" means one or more and thus includes individual components as well as mixtures/combinations.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human lips, skin or eyelashes followed by "kissing" or rubbing a material with the lips, skin or eyelashes, for example, a sheet of paper, after expiration of a certain amount of time following application, such as 2 minutes after application. Similarly, transfer resistance of a composition may be evaluated by the amount of product transferred from a wearer to any other substrate, such as transfer from the lips, skin or eyelashes of an individual to clothing after the expiration of a certain amount of time following application. The amount of composition transferred to the substrate (e.g., clothing or paper) may then be evaluated and compared. For example, a composition may be transfer resistant if a majority of the product is left on the lips, skin or eyelashes. Further, the amount transferred may be compared with that transferred by other compositions, such as commercially available compositions.

"Long wear" compositions as used herein, refers to compositions where at least one property chosen from consistency, texture, and color remains the same as at the time of application, as viewed by the naked eye, after an extended period of time, such as, for example, 1 hour, 2 hours, and further such as 8 hours. Long wear properties may be evaluated by any method known in the art for evaluating such properties. For example, long wear may be evaluated by a test involving the application of a composition to eyelashes and evaluating the consistency, texture and color of the composition after an extended period of time. For example, the consistency, texture and color of a mascara composition may be evaluated immediately following application and these characteristics may then be re-evaluated and compared after an individual has worn the mascara composition for a certain amount of time. Further, these characteristics may be evaluated with respect to other compositions, such as commercially available compositions.

"Waterproof" as used herein refers to the ability to repel water and permanence with respect to water. Waterproof properties may be evaluated by any method known in the art for evaluating such properties. For example, a mascara composition may be applied to false eyelashes, which may then be placed in water for a certain amount of time, such as, for example, 20 minutes. Upon expiration of the pre-ascertained amount of time, the false eyelashes may be removed from the water and passed over a material, such as, for example, a sheet of paper. The extent of residue left on the material may then be evaluated and compared with other compositions, such as, for example, commercially available compositions. Similarly, for example, a composition may be applied to skin, and the skin may be submerged in water for a certain amount of time. The amount of composition remaining on the skin after the pre-ascertained amount of time may then be evaluated and compared. For example, a composition may be waterproof if a majority of the product is left on the wearer, e.g., eyelashes.

"Tackiness" as used herein refers to the adhesion between two substances. For example, the more tackiness there is between two substances, the more adhesion there is between the substances. To quantify "tackiness," it is useful to determine the "work of adhesion" as defined by IUPAC associated with the two substances. Generally speaking, the work of adhesion measures the amount of work necessary to separate two substances. Thus, the greater the work of adhesion associated with two substances, the greater the adhesion there is between the substances, meaning the greater the tackiness is between the two substances.

Work of adhesion and, thus, tackiness, can be quantified using acceptable techniques and methods generally used to measure adhesion, and is typically reported in units of force time (for example, gram seconds ("g s")). For example, the TA-XT2 from Stable Micro Systems, Ltd. can be used to determine adhesion following the procedures set forth in the TA-XT2 Application Study (ref: MATI/PO.25), revised January 2000, the entire contents of which are hereby incorporated by reference. According to this method, desirable values for work of adhesion for substantially non-tacky substances include less than about 0.5 g s, less than about 0.4 g s, less than about 0.3 g s and less than about 0.2 g s. As known in the art, other similar methods can be used on other similar analytical devices to determine adhesion.

According to an embodiment, the cosmetic composition and methods of the present invention may be such that the work of adhesion of the composition is less than about 0.5 g s, less than about 0.4 g s, less than about 0.3 g s and less than about 0.2 g s.

The cosmetic compositions and methods of the present invention can comprise, consist of, or consist essentially of the essential elements and limitations of the invention described herein, as well as any additional or optional ingredients, components, or limitations described herein or any otherwise useful ingredient found in personal care compositions intended for application to keratin materials.

The composition of the present invention may be in any form suitable for use on keratin materials such as, for example, non-solid anhydrous, oil-free or emulsion compositions (for example, water-in-oil emulsion (e.g., water-in-silicone), oil-in-water emulsion (e.g., silicone-in-water), multiple emulsion (e.g., W/O/W or O/W/O), nanoemulsions, etc.). The compositions of the present invention can be eyelash compostions (such as mascaras, topcoats or basecoats), skin compositions (such as foundations, topcoats or basecoats) or lip compositions (such as lipsticks, liquid lip compositions, topcoats or basecoats). Generally speaking, such cosmetic compositions contain colorants such as pigments. Additionally, the compositions of the present invention can be clear or transparent: that is, they can contain little or no colorants. The compositions of the present invention, particularly those with little or no colorants, can be used as a basecoat and/or topcoat for application beneath and/or onto other products applied to keratin materials. Of course, it is to be understood that pigmented compositions can also be used as basecoats or topcoats.

The compositions may be formulated as washable or waterproof. The term washable, as used herein, refers to compositions that generally can be removed with water and/or soap. These formulations are typically emulsions (e.g., of waxes in water) such as creams, or in some cases gels and cakes. Waterproof compositions, on the other hand, typically require use of oils for removal. Such compositions generally come in the form of dispersions of waxes in organic solvents, e.g., isododecane and petroleum distillate.

As defined herein, stability is tested by placing the composition in a controlled environment chamber for 8 weeks at 25°C. In this test, the physical condition of the sample is inspected as it is placed in the chamber. The sample is then inspected again at 24 hours, 3 days, 1 week, 2 weeks, 4 weeks and 8 weeks. At each inspection, the sample is examined for abnormalities in the composition such as phase separation if the composition is in the form of an emulsion. The stability is further tested by repeating the 8-week test at 4°C, 37°C and/or 45°C, and/or under freeze-thaw conditions. A composition is considered to lack stability if in any of these tests an abnormality that impedes functioning of the composition is observed. The skilled artisan will readily recognize an abnormality that impedes functioning of a composition based on the intended application.

### Polyurethane/poly(meth)acrylate graft copolymer

According to the present invention, compositions comprising at least one polyurethane/poly(meth)acrylate graft copolymer are provided. Suitable polyurethane/poly(meth)acrylate graft copolymers include but are not limited to those disclosed in U.S. patent application publication no. 2004-0136937, the entire contents of which is hereby incorporated by reference.

The polyurethane/poly(meth)acrylate graft copolymers may also be referred to as an interpenetrated polymer network (IPN) of a polyurethane and a poly(meth)acrylate. As used herein, the expression "interpenetrated polymer network" refers to a blend of two interlaced polymers, obtained by simultaneous polymerization and/or crosslinking of two types of monomer, the blend obtained having a single glass transition temperature.

Preferred IPNs include those which are commercially available from the company Air Products under the name Hybridur. A polyurethane/poly(meth)acrylates graft copolymer or IPN that is particularly preferred is in the form of an aqueous dispersion of particles e.g., with a weight-average size of between 90 and 110 nm and a number-average size of about 80 nm. This IPN preferably has a glass transition temperature, Tg, ranging from about -60°C to +100°C. An IPN of this type is available from Air Products under the trade name Hybridur 875 (INCI name: POLYURETHANE-2 (and) POLYMETHYL METHACRYLATE). Polyurethane/poly(meth)acrylics available from Air Products under the names Hybridur X-01602 and X 18693-21 are also preferred.

Preferred IPNs such as those discussed above are disclosed in U.S. patent application publication nos. 2003/0215476, 2004/0136937, 2005/0249763, the entire contents of all of which is hereby incorporated by reference.

According to preferred embodiments, the IPNs are polyurethane/poly(meth)acrylate graft copolymers having the following general structure: 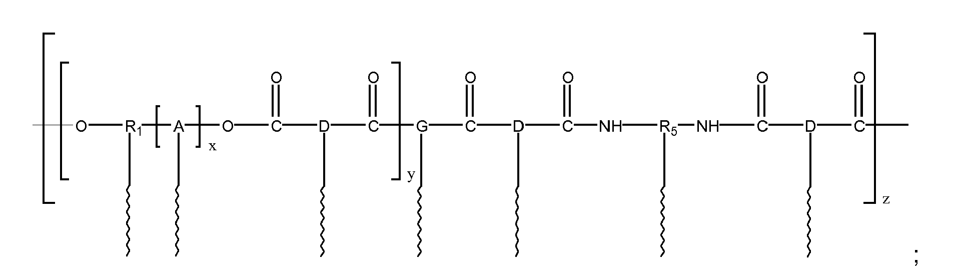 wherein 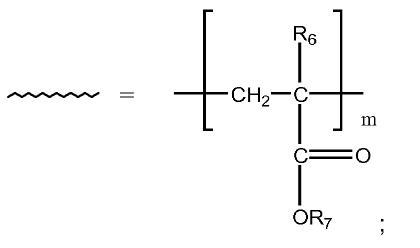 wherein 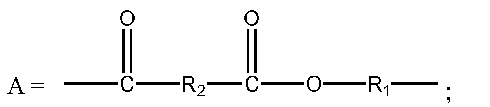 wherein 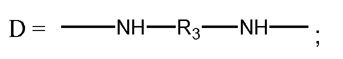 and wherein 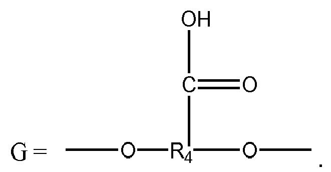 In the formula, R₁, R₂, R₃, R₄, R₅ and R₇ each independently represents an aliphatic hydrocarbon; m represents zero or a positive integer; R₆ represents hydrogen or methyl; and x, y and z each independently represents a positive integer. The graft copolymers may be provided in the form of aqueous dispersions. The graft copolymers may be added to the other components of the composition in powdered form as well.

Preferably, the polyurethane/poly(meth)acrylate graft copolymer is present in an amount sufficient to reduce tackiness of the composition. It is to be understood that the amount of polyurethane/poly(meth)acrylate graft copolymer needed to reduce tackiness of a composition should depend upon the amount of tacky ingredients (for example, tacky film forming agents) present in the composition. For example, the more tacky ingredients which are present, the more polyurethane/poly(meth)acrylate graft copolymer should be needed to reduce tackiness of the composition. In a particularly preferred embodiment, sufficient polyurethane/poly(meth)acrylate graft copolymer is present in the composition to substantially eliminate tackiness of the composition (that is, sufficient polyurethane/poly(meth)acrylate graft copolymer is present in the composition such that one or more characteristics associated with tackiness (for example, stickiness, clumping, poor spreadability, etc.) are not detectible upon application of the composition to a keratin material.

Generally speaking, preferred ranges of polyurethane/poly(meth) acrylate graft copolymer in the composition are from about 0.1 % to about 75% by weight of the total weight of the composition, more preferably from about 0.5% to about 70% of the total weight of the composition, more preferably from about 0.75% to about 50% of the total weight of the composition, more preferably from about 1 % to about 30% of the total weight of the composition, and most preferably from about 1 % to about 15%, including all ranges and subranges therebetween.

### Tackifying film forming agent

According to preferred embodiments of the present invention, the composition further comprises at least one tackifying film forming agent. As used herein, "tackifying film forming agent" means an agent which is capable of forming a film upon application to keratin materials and which provides the composition with tackiness and/or characteristics resulting from tackiness.

Suitable tackifying film forming agents for use in accordance with the present invention include, but are not limited to, polyorganosiloxane containing polymers such as polysilicone polyamides or polysilicone polyurethanes; silicone resins (preferably, MK or MQ resin), silicone/(meth)acrylate copolymers, acrylates/dimethicone copolymers, liquid siloxy silicates and silicone esters such as those disclosed in U.S. Pat. No. 5,334,737, the disclosure of which is hereby incorporated by reference, silicone polymers comprising a backbone chosen from vinyl polymers, methacrylic polymers, and acrylic polymers and at least one chain chosen from pendant siloxane groups and pendant fluorochemical groups, such as those disclosed in U.S. Pat. Nos. 5,209,924 and 4,972,037, and WO 01/32737, the disclosures of which are hereby incorporated by reference in their entirety, a copolymer chosen from vinyl-silicone graft copolymers such as those polymers described in U.S. Pat. No. 5,468,477, the entire disclosure of which is hereby incorporated by reference.

Specific examples of suitable tackifying film forming agents include, but are not limited to, polymers or copolymers comprising monomers selected from the group consisting of acrylic acid, methacrylic acid, acrylate, methacrylate, acrylamide, and mixtures thereof, such as, for example acrylates copolymers (such as, Covacryl A15 and Covacryl E14 by Wackherr), acrylates/ethylhexyl acrylate copolymers (Daitosol 5000SJ by Daito Kasei), butyl acrylate/hydroxypropyl dimethicone acrylate copolymers (Granacrysil BAS by Grant Industries, Inc.), acrylates/C₁₂-C₂₂ alkylmethacrylate copolymers (Allianz OPT by ISP), isododecane and acrylates copolymers (Giovarez AC-5099M by Phoenix), acrylates/octylacrylamide copolymers (Dermacryl-79 by National Starch & Chemical Company), sodium polystyrene sulfonates (Flexan 130 by National Starch & Chemical Company), acrylamides/DMAPA acrylates/methoxy PEG methacrylate copolymer, acrylamide/sodium acrylate copolymer, acrylamide/sodium acryloyldimethyltaurate copolymer, acrylates/acrylamide copolymer, acrylates/t-butylacrylamide copolymers, acrylates/dimethicone copolymer; acrylates/dimethicone methacrylate/ethylhexyl acrylate copolymer; acrylates/dimethylaminoethyl methacrylate copolymer, acrylates/ethylhexyl acrylate copolymer, acrylates/ethylhexylacrylate/HEMA/styrene copolymer, acrylates/hydroxyesters acrylates copolymer, acrylates/laurylacrylate/stearyl acrylate/ethylamine oxide methacrylate copolymer, acrylates/octylacrylamide copolymer, acrylates/propyl trimethicone methacrylate copolymer, acrylates/stearyl acrylate/dimethicone methacrylate copolymer, acrylates/VP copolymer, and acrylates/VP/dimethylaminoethyl methacrylate/diacetone acrylamide/hydroxypropyl acrylate copolymer.

Other suitable tackifying agents include, but are not limited to, aliphatic hydrocarbon resins, aromatic modified aliphatic hydrocarbon resins, hydrogenated polycyclopentadiene resins, polycyclopentadiene resins, gum rosins, gum rosin esters, wood rosins, wood rosin esters, tall oil rosins, tall oil rosin esters, polyterpenes, aromatic modified polyterpenes, terpene phenolics, aromatic modified hydrogenated polycyclopentadiene resins, hydrogenated aliphatic resin, hydrogenated aliphatic aromatic resins, hydrogenated terpenes and modified terpenes, hydrogenated rosin acids, hydrogenated rosin esters, polyisoprene, partially or fully hydrogenated polyisoprene, polybutenediene, partially or fully hydrogenated polybutenediene, and the like.

In some embodiments, the tackifiers can be hydrogenated hydrocarbon resins such as a hydrogenated styrene/methyl styrene/indene copolymer e.g., styrene/methyl styrene/indene copolymers which include R1090, R1100, R7100, S1100, and S51 00, all which are commercially available from Eastman Chemical under the trade name Regalite^{®}. In other embodiments, aliphatic or aromatic hydrocarbon-based tackifying resins, for instance the resins sold under the name "Piccotac" and "Hercotac" from Hercules or "Escorez" from Exxon, may also be used.

In other embodiments, the tackifier can be a copolymer comprising at least one styrene block. For example, a triblock copolymer and in particular those of the polystyrene/polyisoprene or polystyrene/ polybutadiene type, such as those sold or made under the name "Luvitol HSB" by BASF and those of the polystyrene/copoly(ethylene-propylene) type or alternatively of the polystyrene/copoly(ethylene/butylene) type, such as those sold or made under the brand name "Kraton" by Shell Chemical Co. or Gelled Permethyl 99A by Penreco, may be used. Styrene-methacrylate copolymers can be used.

More specifically, mention may be made, for example, of Kraton (G1650 (SEBS), Kraton G1651 (SEBS), Kraton G1652 (SEBS), Kraton G1657X (SEBS), Kraton G1701X (SEP), Kraton G1702X (SEP), Kraton G1726X (SEB), Kraton G1750X (EP) multiarm, Kraton G1765X (EP) multiarm, Kraton D-1101 (SBS), Kraton D-1102 (SBS), Kraton D-1107 (SIS), Gelled Permethyl 99A - 750, Gelled Permethyl 99A- 753-58 (mixture of starburst block polymer and triblock polymer), Gelled Permethyl 99A- 753-59 (mixture of starburst block polymer and triblock polymer), Versagel 5970 and Versagel 5960 from Penreco (mixture of starburst polymer and triblock polymer in isododecane), and OS 129880, OS 129881 and OS 84383 from Lubrizol (styrene-methacrylate copolymer).

Di or triblocks such as polystyrene-copoly(ethylene/propylene) or polystyrene-copoly(ethylene/butylene) such as those described in patent applications WO 98/38981 and US 2002/0055562, the disclosures of which are hereby incorporated by reference, can also be tackifiers in accordance with the present invention.

Suitable tackifying film forming agents for use in accordance with the present invention are also disclosed in U.S. patent application publication no. 2004/0170586, the entire contents of which is hereby incorporated by reference.

According to preferred embodiments, the tackifying film forming agent(s) is/are present in the composition in an amount ranging from 0.3% to 50% by weight relative to the total weight of the composition. Preferably, the film forming agent is present in an amount ranging from 1.0 to 40% by weight relative to the total weight of the composition, and more preferably from 2% to 30%. One of ordinary skill in the art will recognize that these weight percentages are based on dry weight of the film forming agents, and that the film forming agents of the present invention may be commercially available from suppliers in the form of a dilute solution. The amounts of the film forming agent disclosed herein therefore reflect the weight percent of active material.

According to preferred embodiments, the ratio of the tackifying film forming agent to the polyurethane/poly(meth)acrylate graft copolymer present in the composition ranges from about 99.9 : 0.1 to about 0.1 : 99.9, preferably from about 70:30 to about 99:1, and more preferably from about 13:87 to about 87:13.

According to an embodiment, the ratio of acrylates/ethylhexyl acrylate copolymer to the polyurethane/poly(meth)acrylate graft copolymer present in the composition ranges from about 70:30 to about 99:1, and more preferably from about 13:87 to about 87:13.

### Aqueous Dispersions of Polymer Particles

According to preferred embodiments, in addition to the at least one polyurethane/poly(meth)acrylate graft copolymer, the composition can further comprise an aqueous dispersion of polymer particles. Suitable aqueous dispersions of polymer particles are disclosed in U.S. patent application publication no. 2006/0093568, the entire contents of which is hereby incorporated by reference. Preferred aqueous dispersions of polymer particles include ethylene.styrene/acrylates copolymers such as the Syntran series available from Interpolymer, particularly Syntran 5760, and the acrylates/ammonium methacrylate copolymer with tradename Ultrasol 2075C available from Presperse.

According to one embodiment, a composition of the invention may comprise an aqueous dispersion of polymer particles selected from the group consisting of ethylene.styrene/acrylates copolymers, acrylates/ammonium methacrylate copolymer, and mixtures thereof.

In fact, according to one embodiment of the present invention, the polyurethane/poly(meth)acrylate graft copolymer can be replaced by the ethylene.styrene/acrylates copolymers and/or the the acrylates/ammonium methacrylate copolymer in aqueous dispersion. Such preferred aqueous dispersions of polymer particles can detackify a composition in the absence of at least one polyurethane/poly(meth)acrylate graft copolymer.

The aqueous dispersion may be prepared by a person skilled in the art on the basis of his or her general knowledge, especially by emulsion polymerization or by dispersion of the preformed polymer.

Among the film-forming polymers which may be used in aqueous dispersions according to the present invention, mention may be made of synthetic polymers, of polycondensate type or of free-radical type, polymers of natural origin, and mixtures thereof.

Among the polycondensates, mention may thus be made of anionic, cationic, nonionic or amphoteric polyurethanes, polyurethane-acrylics, polyurethane-poly-vinylpyrrolidones, polyester-polyurethanes, polyether-polyurethanes, polyureas, polyurea-polyurethanes, and mixtures thereof.

The polyurethanes may be, for example, an aliphatic, cycloaliphatic or aromatic polyurethane, polyurea/polyurethane or polyurea copolymer, containing, alone or as a mixture.
at least one block of linear or branched aliphatic and/or cycloaliphatic and/or aromatic polyester origin, and/or
at least one block of aliphatic and/or cycloaliphatic and/or aromatic polyether origin, and/or
at least one substituted or unsubstituted, branched or unbranched silicone block, for example polydimethylsiloxane or polymethylphenylsiloxane, and/or

Aat least one block comprising fluoro groups.

The polyurethanes as defined in the invention may also be obtained from branched or unbranched polyesters or from alkyds containing mobile hydrogens, which are modified by means of a polyaddition with a diisocyanate and a difunctional organic co-reactive compound (for example dihydro, diamino or hydroxyamino), also containing either a carboxylic acid or carboxylate group, or a sulphonic acid or sulphonate group, or alternatively a neutralizable tertiary amine group or a quaternary ammonium group.

Mention may also be made of polyesters, polyesteramides, fatty-chain polyesters, polyamides and epoxy ester resins.

The polyesters may be obtained, in a known manner, by polycondensation of aliphatic or aromatic diacids with aliphatic or aromatic diols or with polyols. Succinic acid, glutaric acid, adipic acid, pimelic acid, suberic acid or sebacic acid may be used as aliphatic diacids. Terephthalic acid or isophthalic acid, or alternatively a derivative such as phthalic anhydride, may be used as aromatic diacids. Ethylene glycol, propylene glycol, diethylene glycol, neopentyl glycol, cyclohexanedimethanol and 4,4-N-(1-methylpropylidene)bisphenol may be used as aliphatic diols. Glycerol, pentaerythritol, sorbitol and trimethylolpropane may be used as polyols.

The polyesteramides may be obtained in a similar manner to the polyesters, by polycondensation of diacids with diamines or amino alcohols. Ethylenediamine, hexamethylenediamine or meta- or para-phenylenediamine may be used as diamine. Monoethanolamine may be used as amino alcohol.

As monomer bearing an anionic group which may be used during the polycondensation, mention may be made, for example, of dimethylolpropionic acid, trimellitic acid or a derivative such as trimellitic anhydride, the sodium salt of pentanediol-3-sulphonic acid and the sodium salt of 5-sulpho-1,3-benzenedicarboxylic acid. The fatty-chain polyesters may be obtained using fatty-chain diols during the polycondensation. The epoxy ester resins may be obtained by polycondensation of fatty acids with a condensate having .alpha.,.omega.-diepoxy ends.

The free-radical polymers may in particular be acrylic and/or vinyl polymers or copolymers. Anionic radical polymers are preferred. As a monomer bearing an anionic group which may be used during the free-radical polymerization, mention may be made of acrylic acid, methacrylic acid, crotonic acid, maleic anhydride or 2-acrylamido-2-methylpropanesulphonic acid.

The acrylic polymers may result from the copolymerization of monomers selected from the esters and/or amides of acrylic acid or of methacrylic acid. As examples of monomers of ester type, mention may be made of methyl methacrylate, ethyl methacrylate, butyl methacrylate, isobutyl methacrylate, 2-ethylhexyl methacrylate and lauryl methacrylate. As examples of monomers of amide type, mention may be made of N-t-butylacrylamide and N-t-octylacrylamide.

Acrylic polymers obtained by copolymerization of ethylenically unsaturated monomers containing hydrophilic groups, preferably of nonionic nature, such as hydroxyethyl acrylate, 2-hydroxypropyl acrylate, hydroxyethyl methacrylate and 2-hydroxypropyl methacrylate, are preferably used.

The vinyl polymers may result from the homopolymerization or copolymerization of monomers selected from vinyl esters, styrene or butadiene. As examples of vinyl esters, mention may be made of vinyl acetate, vinyl neodecanoate, vinyl pivalate, vinyl benzoate and vinyl t-butylbenzoate.

Acrylic/silicone copolymers or even nitrocellulose/acrylic copolymers may also be used.

The polymers of natural origin, which are optionally modified, may be selected from shellac, sandarac gum, dammar resins, elemi gums, copal resins, cellulose derivatives, and mixtures thereof.

Mention may also be made of the polymers resulting from the free-radical polymerization of one or more free-radical monomers inside and/or partially at the surface of preexisting particles of at least one polymer selected from the group consisting of polyurethanes, polyureas, polyesters, polyesteramides and/or alkyds. These polymers are generally referred to as "hybrid polymers".

The size of the polymer particles in aqueous dispersion may be between 10 and 500 nm and is preferably between 20 and 150 nm, allowing the production of a film of noteworthy gloss. However, particle sizes ranging up to 1 micron may be used.

Preferably, the aqueous dispersion of polymer particles is present in the composition in an amount ranging are from about 0.1 % to about 75% by weight (active material) of the total weight of the composition, more preferably from about 0.5% to about 70% of the total weight of the composition, more preferably from about 0.75% to about 50% of the total weight of the composition, more preferably from about 1% to about 30% of the total weight of the composition, and most preferably from about 1% to about 15%, including all ranges and subranges therebetween.

### Coloring Agents

According to the present invention, the compositions may optionally comprise at least one coloring agent. Suitable coloring agents include but are not limited to pigments, dyes, such as liposoluble dyes, nacreous pigments, and pearling agents. Typically, when the composition contains colorants, it is a color cosmetic composition such as a foundation, lipstick or mascara. Alternatively, when the composition does not contain colorants or contains very little colorant, it is a clear or transparent composition which can be used as a basecoat (or topcoat) prior to (or after) application of a color cosmetic composition. However, it is possible that topcoats or basecoats could contain colorants, and/or that a mascara composition could contain little or no colorant.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow. The liposoluble dyes, when present, generally have a concentration ranging up to 20% by weight of the total weight of the composition, such as from 0.0001 % to 6%.

The nacreous pigments which may be used according to the present invention may be chosen from white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride. The nacreous pigments, if present, be present in the composition in a concentration ranging up to 50% by weight of the total weight of the composition, such as from 0.1 % to 20%, preferably from 0.1 % to 15%.

The pigments, which may be used according to the present invention, may be chosen from white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium, strontium, calcium, and aluminum.

If present, the pigments may be present in the composition in a concentration ranging up to 50 % by weight of the total weight of the composition, such as from 0,5% to 40%, and further such as from 2% to 30%. In the case of certain products, the pigments, including nacreous pigments, may, for example, represent up to 50% by weight of the composition.

According to particularly preferred embodiments, the compositions of the present invention are in the form of an emulsion. Suitable emulsion forms include but are not limited to oil-in-water, water-in-oil, oil-in-water-in-oil, water-in-oil-in-water and nanoemulsions (emulsions whose oil globules are of very fine particle size, that is to say that they have a number-average size of less than about 100 nanometers (nm)). Emulsions contain at least one oil phase and at least one aqueous phase. Typically speaking, emulsions contain surfactants or surfactant-like materials which provide stability to the emulsions and inhibit de-phasing of the emulsions.

### Additional Ingredients

The compositions of the present invention can also comprise any additive usually used in the field under consideration. For example, film forming agents, dispersants, antioxidants, essential oils, preserving agents, fragrances, liposoluble polymers that are dispersible in the medium, fillers, neutralizing agents, cosmetic and dermatological active agents such as, for example, emollients, moisturizers, vitamins, anti-wrinkle agents, essential fatty acids, sunscreens, and mixtures thereof can be added. A non-exhaustive listing of such ingredients can be found in U.S. patent application serial no. 10/733,467, filed December 12, 2003, the entire contents of which is hereby incorporated by reference. Further examples of suitable additional components can be found in the other references which have been incorporated by reference in this application, including but not limited to the applications from which this application claims priority. Still further examples of such additional ingredients may be found in the International Cosmetic Ingredient Dictionary and Handbook (9th ed. 2002).

A person skilled in the art will take care to select the optional additional additives and/or the amount thereof such that the advantageous properties of the composition according to the invention are not, or are not substantially, adversely affected by the envisaged addition.

These substances may be selected variously by the person skilled in the art in order to prepare a composition which has the desired properties, for example, consistency or texture.

These additives may be present in the composition in a proportion from 0% to 99% (such as from 0.01% to 90%) relative to the total weight of the composition and further such as from 0.1 % to 50% (if present).

Needless to say, the composition of the invention should be cosmetically or dermatologically acceptable, i.e., it should contain a non-toxic physiologically acceptable medium and should be able to be applied to the eyelashes of human beings. For the purposes of the invention, the expression "cosmetically acceptable" means a composition of pleasant appearance, odor, feel and/or taste.

Specific examples of additional ingredients include oils, particularly if the composition is an anhydrous composition or an emulsion. Any oils can be used in accordance with the present invention. The oils can be volatile or non-volatile, silicone-based and/or hydrocarbon-based, etc. Thus, for example, the external oil phase may contain, independently or in combination, volatile silicone oils, non-volatile silicone oils, volatile non-silicone oils and non-volatile non-silicone oils.

In one embodiment, the compositions of the present invention are substantially free of silicone oils (i.e., contain less than about 1 % of silicone oil). In another embodiment, the compositions are substantially free of non-silicone oils (i.e., contain less than about 1% of non-silicone oil). In another embodiment, the compositions are substantially free of non-volatile oils (i.e., contain less than about 1 % of non-volatile oil). In yet another embodiment, the compositions are substantially free of volatile oils (i.e., contain less than about 1 % of volatile oil).

According to one embodiment, the oil phase may contain one or more volatile silicone oils. Examples of such volatile silicone oils include linear or cyclic silicone oils having a viscosity at room temperature less than or equal to 6cSt and having from 2 to 7 silicone atoms, these silicones being optionally substituted with alkyl or alkoxy groups of 1 to 10 carbon atoms. Suitable oils that may be used in the invention include octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

**Table 1**

| Compound | Flash Point (°C) | Viscosity (cSt) |
|---|---|---|
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 (1.5cSt) from Dow Corning | 56 | 1.5 |
| PDMS DC 200 (2cSt) from Dow Corning | 87 | 2 |
| PDMS DC 200 (5cSt) from Dow Corning | 134 | 5 |
| PDMS DC 200 (3St) from Dow Corning | 102 | 3 |

Further, a volatile linear silicone oil may be employed in the compositions of the present invention. Suitable volatile linear silicone oils include those described in U.S. patent no. 6,338,839 and WO03/042221, the contents of which are incorporated herein by reference. In one embodiment the volatile linear silicone oil is decamethyltetrasiloxane. In another embodiment, the decamethyltetrasiloxane is further combined with another solvent that is more volatile than decamethyltetrasiloxane.

The volatility of the solvents/oils can be determined using the evaporation speed as set forth in U.S. patent no. 6,338,839.

Examples of other silicone oils that may be used in the invention include non-volatile linear polydimethylsiloxanes (PDMSs), that are liquid at room temperature; polydimethylsiloxanes comprising alkyl, alkoxy or phenyl groups, which are pendent and/or at the end of a silicone chain, these groups each containing from 2 to 24 carbon atoms; phenylsilicones, for instance phenyl trimethicones, phenyl dimethicones, phenyl trimethylsiloxydiphenylsiloxanes, diphenyl dimethicones, diphenyl methyldiphenyl trisiloxanes and 2-phenylethyl trimethylsiloxysilicates.

According to other preferred embodiments, the oil phase may contain one or more non-silicone volatile oils and may be selected from volatile hydrocarbon oils, alcohols, volatile esters and volatile ethers. Examples of such volatile non-silicone oils include, but are not limited to, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar or Permethyl, the C₈ to C₁₆ branched esters such as isohexyl or isodecyl neopentanoate and their mixtures. Preferably, the volatile non-silicone oils have a flash point of at least 40°C.

**Table 2**

| **Compound** | **Flash Point (°C)** |
|---|---|
| Isododecane | 43 |
| Isohexadecane | 102 |
| Isodecyl Neopentanoate | 118 |
| Propylene glycol n-butyl ether | 60 |
| Ethyl 3-ethoxypropionate | 58 |
| Propylene glycol methylether acetate | 46 |
| Isopar L (isoparaffin C₁₁-C₁₃) | 62 |
| Isopar H (isoparaffin C₁₁-C₁₂) | 56 |

Examples of other non-silicone oils which can be used in the compositions of the present invention include polar oils such as:
- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810, 812 and 818 by the company Dynamit Nobel;
- synthetic oils or esters of formula R₅COOR₆ in which R₅ represents a linear or branched higher fatty acid residue containing from 1 to 40 carbon atoms, including and better still from 7 to 19 carbon atoms, and R₆ represents a branched hydrocarbon-based chain containing from 1 to 40 carbon atoms, including and better still from 3 to 20 carbon atoms, with R₆ + R₇ ≥ 10, such as, for example, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate; and pentaerythritol esters;
- synthetic ethers containing from 10 to 40 carbon atoms;
- C₈ to C₂₆ fatty alcohols, for instance oleyl alcohol; and
- mixtures thereof.

Preferably, the oils, if present, represent from about 5% to about 80% by weight of the total weight of the composition, more preferably from about 10% to about 60% of the total weight of the composition, and most preferably from about 15% to about 50%, including all ranges and subranges therebetween.

According to preferred embodiments, the compositions of the present invention may further comprise at least one wax. For the purposes of the present invention, the term "wax" means a lipophilic fatty compound that is solid at room temperature (25°C) and atmospheric pressure (760 mmHg, *i.e.* 10⁵ Pa), which undergoes a reversible solid/liquid change of state and which has a melting point of greater than 30°C and in some embodiments, greater than 55 °C up to 120°C or even as high as 200 °C. Suitiable waxes include those which are generally used in cosmetics and dermatology. A variety of waxes may be useful, including waxes of animal origin, waxes of plant origin, waxes of mineral origin and waxes of synthetic origin. Examples of waxes of animal origin include beeswaxes, lanolin waxes and Chinese insect waxes. Examples of waxes of plant origin include rice waxes, carnauba wax, candellila wax and ouricurry wax, cork fibre waxes, sugar cane waxes, Japan waxes, sumach wax and cotton wax. Examples of waxes of mineral origin include paraffins, microcrystalline waxes, montan waxes and ozokerites. Examples of waxes of synthetic origin include polyolefin waxes, e.g., polyethylene waxes, waxes obtained by Fischer-Tropsch synthesis, waxy copolymers and their esters, and silicone and fluoro waxes. Alternatively, hydrogenated oils of animal or plant origin may be used. Examples include hydrogenated jojoba waxes and hydrogenated oils which are obtained by catalytic hydrogenation of fats composed of a C₈-C₃₂ linear or nonlinear fatty chain, hydrogenated sunflower oil, hydrogenated castor oil, hydrogenated copra oil, hydrogenated lanolin and hydrogenated palm oils. In some embodiments, the compositions contain at least two waxes.

In some embodiments, the wax may have a low melting point, generally ranging from about 35-70 °C, but unlike such low MP waxes, has a hardness (e.g., generally about 7-10 mm/10, as measured in accordance with ASTM-D1321) that is associated with waxes having high melting points. Such waxes are commercially available from Mineral and Pigment Solutions, Inc. or Koster Keunen under the name BK, e.g., BK-42 (INCI name: tetradecyl-octadecanyl-behenate; CAS No.:231627-84-2; C₅₄H₁₀₈O₂) and BK-60 (INCI name:hexadecyl-cosanyl-hecacosanate; C₆₂H₁₂₄O₂).

The wax, if present, may be in the compositions in an amount generally ranging from about 0.1 % to about 50%, preferably from about 0.5% to about 20%, and more preferably from about 1 % to about 10% by weight, relative to the total weight of the composition.

According to other embodiments, the compositions may be wax-free (containing no wax) or substantially wax free (containing less than 0.1 % wax).

Water, when present, preferably represents from about 5% to about 90% by weight of the total weight of the composition, more preferably from about 6% to about 40% of the total weight of the composition, more preferably from about 7% to about 70% of the total weight of the composition, more preferably from about 8% to about 60% of the total weight of the composition, and most preferably from about 10% to about 50%, including all ranges and subranges therebetween.

Of course, the compositions of the present invention can be substantially anhydrous (that is, they can comprise less than 5% water), essentially anhydrous (that is, they can comprise less than 2% water), anhydrous (that is, they can comprise less than 1% water, or water-free (that is, they contain no water).

In addition to water, the aqueous phase of the composition, if present, may contain a water-miscible solvent (miscibility in water of greater than 50% by weight at 25 °C), for instance, lower mono-alcohols containing from 1 to 5 carbon atoms such as ethanol or isopropanol, glycols containing from 2 to 8 carbon atoms, such as propylene glycol, ethylene glycol, butylene glycol or dipropylene glycol, C3-C4 ketones and C2-C4 aldehydes. The aqueous phase may be present in a content ranging from about 1 % to about 95% by weight, relative to the total weight of the composition, in some embodiments from about 3% to about 70% by weight, and more preferably from about 5% to about 45%, 50%, 55% or 60% by weight.

The compositions of the invention may contain surfactants. Surfactants typically employed in the compositions of the present invention include anionic, nonionic and cationic surfactants. See, *e.g.,* Encyclopedia of Chemical Technology, KIRK-OTHMER, volume 22, pp. 333-432, 3rd edition, 1979, Wiley, for the definition of the properties and (emulsifying) functions of the surfactants, in particular pp. 347-377 of this publication regarding anionic and nonionic surfactants. Examples of surfactants useful in the compositions of the invention are include as nonionic surfactants, fatty acids, fatty alcohols, polyethoxylated fatty alcohols or polyglycerolated fatty alcohols, such as polyethoxylated stearyl alcohols or cetylstearyl alcohols, esters of fatty acid and sucrose, and glucose alkyl esters, in particular polyoxyethylenated C₁ - C₆ alkyl glucose fatty esters, and as anionic surfactants, C₁₆ - C₃₀ fatty acids neutralized by amines, ammonia or the alkali metal salts thereof. Examples of cationic surfactants include quaternary amines, amine oxides and amines, *e.g.,* alkyl amines, alkyl imidazolines, ethoxylated amines, quaternary compounds, and quaternized esters. Cationic surfactants may also provide a conditioning effect.

Surfactants are generally present in amounts ranging from about 1 to about 30% by weight, and in some other embodiments from about 3% to about 15% by weight, relative to the total weight of the composition.

The compositions of the invention may contain fibers. Suitable fibers may be chosen from natural and synthetic fibers. Natural fibers include, but are not limited to, cotton, silk, wool, and other keratin fibers. Synthetic fibers include, but are not limited to, polyester, rayon, nylon and other polyamide fibers. In one embodiment, the fibers of the invention are chosen from elastomeric fibers, such as those described in EP 1172 078 A2, more specifically paragraphs 16-24, the disclosure of which is herein incorporated by reference.

Fibers, if present, may be present in the composition in an amount ranging from 0.5% to 10% relative to the total weight of the composition. In a further embodiment, the fibers are present in an amount ranging from 1% to 5% relative to the total weight of the composition. In one embodiment, the fibers may, for example, have an average length ranging from 0.5 mm to 4.0 mm, such as from 1.5 mm to 2.5 mm.

The compositions of the present invention may also comprise at least one polysaccharide resin. Suitable polysaccharide resins comprise numerous hydroxyl groups and hydrophobic groups. The at least one polysaccharide resin can be in the form of colloidal suspensions of fine, highly modified particles such as starch particles. The fine particles may vary in size, and may, for example, include particles with a diameter of 10 microns or less.

Non-limiting examples of the at least one polysaccharide resin include the polysaccharide resins available from KAMA, International Corp., Duluth, Ga. For example, polysaccharide resin KM13 is a highly modified, colloidal suspension in water of finely divided starch particles with a diameter of less than 10 microns. KM13 is a co-reactive resin which will form hydrogen bonds with other resins. This polysaccharide resin contains numerous hydroxyl groups which contribute to the wetting of pigments in aqueous systems and hydrophobic groups that permit acceptance in solvent based systems without pigment flocculation or flotation.

Depending upon the nature of the inventive composition, controlling viscosity may be important from the standpoints of fast and easy application of the composition, as well as uniform coating. In the case of washable and waterproof mascara, for example, viscosity of washable mascara generally ranges from about 5 to about 80 pascal seconds (Pa.s), and preferably from about 10 to about 40 Pa.s, and viscosity of waterproof mascara generally ranges from about 5 to about 80 Pa.s, and preferably from about 10 to about 40 Pa.s. Viscosity is measured at 25 °C with a Rheomat RM 180 viscometer fitted with a No. 4 rotor, wherein the measurement is carried out after spinning the rotor for 10 minutes (after which time stabilization of the viscosity and of the rotor spin speed are observed), at a shear rate of 200 s⁻¹.

Viscosity may be adjusted by adding a thickener. Representative examples include cellulose-based thickeners, for example, water-soluble cellulose-based thickeners, such as hydroxyethylcellulose, methylcellulose, hydroxypropylcellulose and carboxymethylcellulose. Among these thickeners, specific examples include alginates, maltodextrin, polysaccharide resins such as starch and its derivatives, hyaluronic acid and its salts, clays, and, in particular, montmorillonites, hectorites and Iaponites, crosslinked polyacrylic acids, such as the "Carbopol" products from the company Goodrich, the polyglyceryl (meth)acrylate polymers sold under the names "Hispagel" or "Lubragel" by the companies Hispano Quimica or Guardian, polyvinylpyrrolidone (PVP), polyvinyl alcohol, crosslinked acrylamide polymers and copolymers, such as those sold under the names "PAS 5161" or "Bozepol C" by the company Hoechst, "Sepigel 305" by the company SEPPIC, crosslinked methacryloyloxyethyltrimethylammonium chloride homopolymers sold under the name "Salcare SC95" by the company Allied Colloid, and associative polymers and, in particular, associative polyurethanes. Oil soluble thickening agents may also be used. See, U.S. Patent Publications 2003/0215413, 2005/0065046 and 2002/0028226.

The thickening agent, if present, is generally present in an amount ranging from about 0.1 % to about 20% by weight, and preferably from about 0.5% to about 10% by weight.

According to preferred embodiments of the present invention, methods of improving performance properties of compositions for keratin materials (for example, skin, lips or eyelashes) such as, for example, transfer-resistance, long-wear and/or waterproofing, comprising adding at least one polyurethane/poly(meth)acrylate graft copolymer to the composition are provided. The compositions may be applied to the keratin materials as needed, preferably once or twice daily, more preferably once daily and then preferably allowed to dry before subjecting to contact such as with clothing or other objects.

According to other preferred embodiments, methods of increasing eyelash volume and/or length comprising applying to eyelashes an eyelash volume- and/or length-increasing effective amount of a composition comprising at least one polyurethane/poly(meth)acrylate graft copolymer are provided.

According to other preferred embodiments, methods of curling eyelashes comprising applying to eyelashes an eyelash curling effective amount of a composition comprising at least one polyurethane/poly(meth)acrylate graft copolymer are provided.

According to yet further embodiments of the present invention, methods of making-up keratin materials such as skin, lips or eyelashes comprising applying a keratin material making-up effective amount of a composition comprising at least one polyurethane/poly(meth)acrylate graft copolymer to keratin materials in need of such making-up are provided.

According to preferred embodiments of the present invention, methods of treating or caring for keratin materials such as skin, lips or eyelashes by applying compositions of the present invention to the keratin materials in an amount sufficient to treat and/or care for the keratin materials are provided.

According to other preferred embodiments, methods of enhancing the appearance of keratin materials such as skin, lips or eyelashes by applying compositions of the present invention to the keratin materials in an amount sufficient to enhance the appearance of the keratin materials are provided.

In accordance with the preceding preferred embodiments, the compositions of the present invention are applied topically to keratin materials in an amount sufficient to treat, care for and/or make up the keratin materials, or to enhance the appearance of the keratin materials. The compositions may be applied to keratin materials as needed, preferably once or twice daily, more preferably once daily and then preferably allowed to dry before subjecting to contact such as with clothing or other objects.

According to other embodiments of the present invention, methods for detackifying a composition comprising adding at least one polyurethane/poly(meth)acrylate graft copolymer to the composition in an amount sufficient to reduce the tackiness of the composition are provided.

According to yet other preferred embodiments of the present invention, methods of preparing a composition comprising at least one polyurethane/poly(meth)acrylate graft copolymer and at least one tackifying film forming agent comprising dispersing either the at least one polyurethane/poly(meth)acrylate graft copolymer or the at least one tackifying film forming agent in a composition, and then adding the other ingredient to the composition are provided. According to one such preferred embodiment, the at least one polyurethane/poly(meth)acrylate graft copolymer is dispersed in the composition, and then the at least one tackifying film forming agent is added to the composition. According to another such preferred embodiment, the at least one tackifying film forming agent is dispersed in the composition, and then the at least one polyurethane/poly(meth)acrylate graft copolymer is added to the composition.

The present invention also envisages kits and/or prepackaged materials suitable for consumer use containing one or more compositions according to the description herein (for example, kits containing (1) a colored cosmetic product such as a foundation, lip composition or mascara; and (2) a basecoat and/or topcoat). The packaging and application device for any subject of the invention may be chosen and manufactured by persons skilled in the art on the basis of their general knowledge, and adapted according to the nature of the composition to be packaged. Indeed, the type of device to be used can be in particular linked to the consistency of the composition, in particular to its viscosity; it can also depend on the nature of the constituents present in the composition, such as the presence of volatile compounds.

Unless otherwise indicated, all numbers expressing quantities of ingredients, reaction conditions, and so forth used in the specification and claims are to be understood as being modified in all instances by the term "about." Any numerical value, however, inherently contain certain errors necessarily resulting from the standard deviation found in their respective measurements. Accordingly, unless indicated to the contrary, the numerical parameters set forth in the following specification and attached claims are approximations that may vary depending upon the desired properties sought to be obtained by the present invention.

The following examples are intended to illustrate the invention without limiting the scope as a result. The percentages are given on a weight basis.

**Example 1 - Waterproof Mascara**

| Trade Name | INCI Name | |
|---|---|---|
| | | |
| Isododecane | Isododecane | 43 |
| Regalite 1100 | Hydrogenated Styrene/ Methyl styrene/ Indene Copolymer | 19 |
| Kraton G 1657 M | Hydrogenated Styrene/Butadiene Copolymer | 5 |
| Stearyl Polyacrylate | Poly C10-30 Alkyl Acrylate | 2 |
| Bentone | Disteardimonium Hectorite | 6 |
| LAN Phase | See composition below | 2 |
| Jeesilc IDD | Cyclized Polydimethyl Siloxane in Isododecane Solvent | 8 |
| Propylene Carbonate | Propylene Carbonate | 3 |
| Tospearl | | 4 |
| Mica | Mica | 4 |
| Hybridur 875 | Polyurethane-2 (and) Polymethyl Methacrylate | 4 |
| | | |
| **Total** | | **100** |

### Procedure

1. In the main kettle isododecane was heated to 65-75°C. Under mixing, Regalite 1100 was added and dissolved (about 15 minutes).
2. After the solid was completely dissolved, Kraton G 1657 M was added under vigorous mixing.
   The temperature was maintained between 65 and 75°C.
3. After all solids were dissolved, the composition was removed from heat.
4. Stearyl polyacrylate was added and mixed well until dissolved.
5. Bentone was added and mixed well until dissolved.
6. The LAN phase, Jeesilc IDD, Tospearl and Mica were added and mixed well.
7. Hybridur 875 was added and mixed well.
8. When the temperature reached 55-60°C, propylene carbonate was added and mixed well.
9. All ingredients were mixed until well mixed.

**Composition of LAN Phase**

| | |
|---|---|
| 0.7% | Phenoxyethanol |
| 0.3% | Disodium EDTA |
| 15.0% | Sodium Glycolate |
| 6.0% | Octylacrylamide/Acrylates/Butylaminoethylmethacrylate Copolymer |
| 0.5% | Tocopherol Acetate |
| 5.0% | Lecithin |
| 1.0% | Simethicone |
| 20.0% | Isoceteth-20 |
| 0.1% | Propylparaben |
| 0.1% | Methylparaben |
| 51.3% | Water |

| Trade Name | INCI Name | |
|---|---|---|
| | | |
| Isododecane | Isododecane | 51 |
| Regalite 1100 | Hydrogenated Styrene/ Methyl styrene/ Indene Copolymer | 19 |
| Kraton G 1657 M | Hydrogenated Styrene/ Butadiene Copolymer | 8 |
| Puresyn 150 | Hydrogenated Polydecene | 0.8 |
| PolyStearyl Acrylate | Poly C10-30 Alkyl Acrylate | 5 |
| Bentone | Disteardimonium Hectorite | 4 |
| LAN Phase | See composition below | 2 |
| EL-8040 ID | | 2 |
| Hybridur 875 | Polyurethane-2 (and) Polymethyl Methacrylate | 3 |
| Syntran 5760 | Styrene/Acrylates/Ammonium Methacrylate Copolymer (and) Butylene Glycol (and) Sodium Laureth-12 Sulfate | 3 |
| Silica | Silica | 1 |
| Propylene Carbonate | Propylene Carbonate | 1.2 |
| | | |
| **Total** | | **100** |

### Example 2 - Waterproof Mascara

### Procedure

1. In the main kettle isododecane was heated to 65-75°C. Under mixing, Regalite 1100 was added and dissolved (about 15 minutes).
2. After the solid was completely dissolved, Kraton G 1657 M was added under vigorous mixing.
   The temperature was maintained between 65 and 75°C.
3. After all solids were dissolved, the composition was removed from heating.
4. Puresyn 150 was added and mixing was continued.
5. PolyStearyl Acrylate and Bentone were added and mixed well until dissolved.
6. LAN Phase, Hybridur 875, Syntran 5760, and Silica were added and mixed well.
7. When the temperature reached 55-60°C, propylene carbonate was added and mixed well.

**Composition of LAN Phase**

| | |
|---|---|
| 0.7% | Phenoxyethanol |
| 0.3% | Disodium EDTA |
| 15.0% | Sodium Glycolate |
| 6.0% | Octylacrylamide/Acrylates/Butylaminoethylmethacrylate Copolymer |
| 0.5% | Tocopherol Acetate |
| 5.0% | Lecithin |
| 1.0% | Simethicone |
| 20.0% | Isoceteth-20 |
| 0.1% | Propylparaben |
| 0.1% | Methylparaben |
| 51.3% | Water |

### Examples 3-4

The washable mascaras in Examples 3-4 were prepared via the following general procedure:
- 1.: Melt Beeswax and Carnauba Wax (**Phase A1**) at 75-80°C and start homogenizing (water bath should be at about 85°C)
- 2.: In separate beaker combine water and carbon black (**Phase B**), mix and begin heating to 75-80°C.
- 3.: To main beaker, add Methyl Paraben (**Phase A2**) and mix for 5 minutes
- 4.: To main beaker, add PMMA (**Phase A3**) and mix for 15 minutes
- 5.: Add **Phase B to Phase A** and homogenize 15-20 minutes
- 6.: Add Butylene Glycol (**Phase C**) and mix for 5 minutes
- 7.: Still under heat transfer to paddle
- 8.: Add Sodium Polymethacrylate (**Phase D**) and mix for 10 minutes
- 9.: Add Preservative blend (**Phase E**) and mix for 10 minutes
- 10.: Add Sodium Hydroxide (**Phase F**) and mix for 10 minutes
- 11.: Add **Phase G** (pre-mixed with propeller mixer at room temperature), mix 5 minutes and turn off heat.
- 12.: SLOWLY add Diatosol SJ (**Phase H1**) at 55-60°C, mix 10-15 minutes
- 13.: SLOWLY add Hybridur (**Phase H2**) and mix

- 14.: drop batch at room temperature

| | **Example 3** | |
|---|---|---|
| | **INCI NAME** | |
| **A1** | *PEG* 8 *Beeswax* | **22.00** |
| | *Carnauba K82H* | **5.50** |
| | | |
| **A2** | *Methylparaben* | **0.20** |
| | | |
| **A3** | *PMMA* | **2.00** |
| | | |
| **B** | *Carbon Black grind* | **20.00** |
| | *Water* | **10.80** |
| | | |
| **C** | *Butylene Glycol* | **5.00** |
| | | |
| **D** | *Sodium Polymethacrilate* | **1.00** |
| | | |
| **E** | *Phenonip* | **0.70** |
| | | |
| **F** | *NaOH (50% solution)* | **0.20** |
| | | |
| **G** | *Water* | **2.50** |
| | *Polyquaternium-10* | **0.10** |
| | | |
| **H1** | *Diatosol 5000 SJ* | **25.00** |
| | | |
| **H2** | *Hybridur* | **5.00** |

| | | |
|---|---|---|
| **COMPOSITION OF COLORANT FOR EXAMPLE 3** **CARBON BLACK = 20%** **WATER =74.4%** **ACACIA =5%** **METHYLPARABEN = 0.3** **IMIDAZOLIDINYL UREA = 0.3** | | |

| | **Example 4** | |
|---|---|---|
| | **INCI NAME** | |
| **A1** | *PEG* 8 *Beeswax* | **22.00** |
| | *Carnauba (and) PEG-14 Stearate (and) Ceteth 20* | **5.50** |
| | | |
| **A2** | *Preservative* | **0.20** |
| | | |
| **A3** | *Polymethyl Methacrylate (and) Isopropyl Titanium Triisostearate* | **2.00** |
| | | |
| **B** | *Colorant* | **4.00** |
| | *Water* | **26.80** |
| | | |
| **C** | *Butylene Glycol* | **5.00** |
| | | |
| **D** | *Sodium Polymethacrylate* | **1.00** |
| | | |
| **E** | *Preservative* | **0.70** |
| | | |
| **F** | *Sodium Hydroxide* | **0.10** |
| | *Water* | **0.10** |
| | | |
| **G** | *Water* | **2.50** |
| | *Polyquaternium-10* | **0.10** |
| | | |
| **H1** | *AcrylateslEthylhexyl Acrylate Copolymer* | **25.00** |
| | | |
| **H2** | *Polyurethane-2 (and) Polymethyl Methacrylate* | **5.00** |
| | | |
| | | **100.00** |

| | **Example 5** | |
|---|---|---|
| | **INCI NAME** | |
| **A1** | *PEG* 8 *Beeswax* | **22.00** |
| | *Carnauba (and) PEG-14 Stearate (and) Ceteth 20* | **5.50** |
| | | |
| **A2** | *Preservative* | **0.20** |
| | | |
| **A3** | *Polymethyl Methacrylate (and) Isopropyl Titanium Triisostearate* | **2.00** |
| | | |
| **B** | *Colorant* | **2.00** |
| | *Water* | **29.70** |
| | | |
| **C** | *Butylene Glycol* | **4.00** |
| | | |
| **D** | *Sodium Polymethacrilate* | **1.00** |
| | | |
| **F** | *Preservative* | **0.70** |
| | | |
| **G** | *Sodium Hydroxide* | **0.10** |
| | *Water* | **0.10** |
| | | |
| **H** | *Water* | **2.50** |
| | *Polyquaternium-10* | **0.10** |
| | | |
| **I** | *Rayon* | **0.10** |
| | | |
| **J1** | *Acrylates*/*Ethylhexyl Acrylate Copolymer* | **25.00** |
| | | |
| **J2** | *Polyurethane-2 (and) Polymethyl Methacrylate* | **5.00** |
| | | |
| | | **100.00** |

| | | |
|---|---|---|
| **COMPOSITION OF COLORANT FOR EXAMPLE 5** **CARBON BLACK = 20%** **WATER =63.2%** **PROPYLENE GLYCOL=10.0%** **PVP = 5.0%** **PHENOXYETHANOL = 1.0%** **METHYLPARABEN = 0.8%** | | |

| | **Example 6** | |
|---|---|---|
| | **INCI NAME** | |
| **A1** | *PEG* 8 *Beeswax* | **20.00** |
| | *Carnauba (and) PEG-14 Stearate (and) Ceteth 20* | **5.00** |
| | *Preservative* | **0.20** |
| | *Polymethyl Methacrylate (and) Isopropyl Titanium Triisostearate* | **2.00** |
| | | |
| **A2** | *Water* | **41.60** |
| | *Colorant* | **4.00** |
| | *Hydrolyzed Corn Starch* | **1.00** |
| | | |
| **A3** | *Butylene Glycol* | **5.00** |
| | | |
| **A4** | *PEG*/*PPG17*/*18 Dimethicone* | **0.50** |
| | | |
| **B** | *Acrylates*/*Ethylhexyl Acrylate Copolymer* | **12.00** |
| | *Polyurethane-2 (and) Polymethyl Methacrylate* | **8.00** |
| | | |
| **C** | *Preservative* | **0.70** |
| | | **100.00** |

### Example 7 -- Tackiness Measurement

The following two mascara compositions were prepared.

| | **Comparative Composition** | |
|---|---|---|
| | **INCI NAME** | |
| **A1** | *PEG* 8 *Beeswax* | **22.00** |
| | *Carnauba K82H* | **5.50** |
| | | |
| **A2** | *Preservative* | **0.20** |
| | | |
| **A3** | *Polymethyl Methacrylate (and) Isopropyl Titanium Triisostearate* | **2.00** |
| | | |
| **B** | *Carbon Black grind* | **12.00** |
| | *Water* | **23.80** |
| | | |
| **C** | *Butylene Glycol* | **5.00** |
| | | |
| **D** | *Sodium Polymethacrilate* | **1.00** |
| | | |
| **F** | *Preservative* | **0.70** |
| | | |
| **G** | *Sodium Hydroxide* | **0.20** |
| | | |
| **H** | *Water* | **2.50** |
| | *Polyquatemium-10* | **0.10** |
| | | |
| **J1** | *Acrylates*/*Ethylhexyl Acrylate Copolymer* | **25.00** |
| | | |
| | | **100.00** |

| | **Invention Composition** | |
|---|---|---|
| | **INCI NAME** | |
| **A1** | *PEG* 8 *Beeswax* | **22.00** |
| | *Carnauba K82H* | **5.50** |
| | | |
| **A2** | *Preservative* | **0.20** |
| | | |
| **A3** | *Polymethyl Methacrylate (and) Isopropyl Titanium Triisostearate* | **2.00** |
| | | |
| **B** | *Carbon Black grind* | **12.00** |
| | *Water* | **18.80** |
| | | |
| **C** | *Butylene Glycol* | **5.00** |
| | | |
| **D** | *Sodium Polymethacrilate* | **1.00** |
| | | |
| **F** | *Preservative* | **0.70** |
| | | |
| **G** | *Sodium Hydroxide* | **0.20** |
| | | |
| **H** | *Water* | **2.50** |
| | *Polyquaternium-10* | **0.10** |
| | | |
| **J1** | *Acrylates*/*Ethylhexyl Acrylate Copolymer* | **25.00** |
| | | |
| **J2** | *Polyurethane-2 (and) Polymethyl Methacrylate* | **5.00** |
| | | |
| | | **100.00** |

The tackiness of these two compositions was tested as follows. 1.0g of each composition was spread evenly across 2.25" x 6" area on BioSkin material obtained from U.S. Cosmetics Corporation.

A Texture Analyzer TZ-XT2 using 1.5" round, flat probe was covered with the BioSkin cutout. The settings for this instrument were as follows.

### Settings:

Option: Adhesive Test
Pre-Test Speed: 0.1mm/s
Test Speed: 0.1mm/s
Post-Test Speed: 10mm/s
Force: 100g
Distance: 0.5mm
Trigger Value: 0.5g

At room temperature, 10 arbitrary locations along each film were measured, and reliable data points were averaged. Analysis was performed using the procedures set forth in the TA-XT2 Application Study (ref: MATI/PO.25), revised January 2000.

The results demonstrated that the Invention Composition had 23 times less tackiness than the Comparative Composition.

## Claims

1. A method for detackifying a composition comprising adding at least one polyurethane/poly(meth)acrylate graft copolymer to the composition in an amount sufficient to reduce the tackiness of the composition.

2. The method of claim 1, wherein the copolymer is present as an aqueous dispersion of particles.

3. The method of claim 2, wherein said particles have a weight-average size of between about 90 nm and about 110 nm, a number-average size of about 80 nm, and a glass transition temperature ranging from about -60 °C to about +100 °C.

4. The method according to anyone of claims 1 to 3, wherein the copolymer is present in the composition in an amount of about 0.1% to about 75% by weight relative to the total weight of the composition, more preferably from about 0.75% to about 50% by weight relative to the total weight of the composition, and more preferably from about 1% to about 30% by weight relative to the total weight of the composition.

5. The method according to anyone of claims 1 to 4, wherein the composition is an eyelash composition.

6. The method according to anyone of claims 1 to 5, wherein the composition comprises at least one tackifying film forming agent.

7. The method of claim 6, wherein the at least one tackifying film forming agent is a polymer or copolymer comprising monomers selected from the group consisting of acrylic acid, methacrylic acid, acrylate, methacrylate, and mixtures thereof.

8. The method of claim 6 or 7, wherein the at least one tackifying film forming agent is an acrylates/ethylhexyl acrylate copolymer.

9. The method according to anyone of claims 6 to 8, wherein the ratio of tackifying film forming agent to polyurethane/poly(meth)acrylate graft copolymer present in the composition ranges from about 70:30 to about 99:1, and preferably from about 13:87 to about 87:13.

10. The method of claim 8, wherein the ratio of acrylates/ethylhexyl acrylate copolymer to polyurethane/poly(meth)acrylate graft copolymer present in the composition ranges from about 70:30 to about 99:1, and preferably from about 13:87 to about 87:13.

11. The method according to anyone of claims 1 to 10, wherein the work of adhesion of the composition is less than about 0.5 g s, less than about 0.4 g s, less than about 0.3 g s, and less than about 0.2 g s.

12. The method according to anyone of claims 1 to 11, wherein the composition further comprises an aqueous dispersion of polymer particles selected from the group consisting of ethylene.styrene/acrylates copolymers, acrylates/ammonium methacrylate copolymers, and mixtures thereof.

13. The method according to anyone of claims 1 to 12, wherein the composition further comprises at least one coloring agent.

14. A composition comprising at least one polyurethane/poly(meth)acrylate graft copolymer and at least one tackifying film forming agent, wherein the polyurethane/poly(meth)acrylate graft copolymer is present in an amount sufficient to reduce the tackiness of the composition.

15. The composition of claim 14, wherein the copolymer is present as an aqueous dispersion of particles.

16. The composition of claim 15, wherein said particles have a weight-average size of between about 90 nm and about 110 nm, a number-average size of about 80 nm, and a glass transition temperature ranging from about -60 °C to about +100 °C.

17. The composition according to anyone of claims 14 to 16, wherein the copolymer is present in the composition in an amount of about 0.75% to about 50% by weight relative to the total weight of the composition.

18. The composition according to anyone of claims 14 to 17, wherein the composition is an eyelash composition.

19. The composition according to anyone of claims 14 to 18, wherein the at least one tackifying film forming agent is an acrylates/ethylhexyl acrylate copolymer.

20. The composition according to anyone of claims 14 to 19, wherein the ratio of tackifying film forming agent to polyurethane/poly(meth)acrylate graft copolymer present in the composition ranges from about 70:30 to about 99:1, and preferably from about 13:87 to about 87:13.

21. The composition according to anyone of claims 14 to 20, wherein the composition further comprises an aqueous dispersion of polymer particles selected from the group consisting of ethylene.styrene/acrylates copolymers, acrylates/ammonium methacrylate copolymers, and mixtures thereof.

22. A composition comprising an aqueous dispersion of polymer particles selected from the group consisting of ethylene.styrene/acrylates copolymers, acrylates/ammonium methacrylate copolymers, and mixtures thereof, and at least one tackifying film forming agent, wherein the aqueous dispersion of polymer particles is present in an amount sufficient to reduce the tackiness of the composition.

23. A method of improving transfer-resistance and/or long-wear properties of a composition for a keratin material comprising adding at least one polyurethane/poly(meth)acrylate graft copolymer to the composition in an amount sufficient to improve the transfer-resistance and/or long-wear properties of the composition.

24. A method of preparing a composition comprising at least one polyurethane/poly(meth)acrylate graft copolymer and at least one tackifying film forming agent comprising dispersing either the at least one polyurethane/poly(meth)acrylate graft copolymer or the at least one tackifying film forming agent in a composition, and then adding the other ingredient to the composition.

25. The method of claim 24, wherein the at least one polyurethane/poly(meth)acrylate graft copolymer is dispersed in the composition, and then the at least one tackifying film forming agent is added to the composition.

26. The method of claim 24 , wherein the at least one tackifying film forming agent is dispersed in the composition, and then the at least one polyurethane/poly(meth)acrylate graft copolymer is added to the composition.

27. A method of making the composition of claim 14, comprising dispersing either the at least one polyurethane/poly(meth)acrylate graft copolymer or the at least one tackifying film forming agent in a composition, and then adding the other ingredient to the composition.
